# EUROPEAN PATENT SPECIFICATION

(11) **EP 3 398 444 B1**
(45) Date of publication and mention of the grant of the patent: **03.03.2021**
(21) Application number: 18174952.4
(22) Date of filing: 20.06.2013
(51) Int. Cl.: A23J 3/00, A61K 35/64, C11B 1/02, C11B 1/10, A23J 3/34, A23L 33/00, A23L 35/00, A23K 20/147, A23K 20/158, A23D 9/04

(54) **METHOD TO CONVERT INSECTS OR WORMS INTO NUTRIENT STREAMS AND COMPOSITIONS OBTAINED THEREBY**
VERFAHREN ZUR UMWANDLUNG VON INSEKTEN ODER WÜRMERN IN NÄHRSTOFFSTRÖME UND DADURCH GEWONNENE ZUSAMMENSETZUNGEN
PROCÉDÉ POUR CONVERTIR DES INSECTES OU DES VERS EN FLUX DE NUTRIMENTS ET COMPOSITIONS AINSI OBTENUES

(30) Priority: 21.06.2012 NL 2009044; 21.06.2012 US 201261662420 P
(43) Date of publication of application: 07.11.2018
(62) Divisional of application: 13737445.0
(73) Proprietor: Bühler Insect Technology Solutions AG, 9240 Uzwil (CH)
(72) Inventor: Arsiwalla, Tarique, 5107 NC Dongen (NL); Aarts, Kees Wilhelmus Petrus, 5107 NC Dongen (NL)
(74) Representative: Vossius & Partner Patentanwälte Rechtsanwälte mbB

(56) References cited:
- CN-A- 1 297 691
- CN-A- 1 415 757
- CN-A- 102 048 020
- CN-A- 102 422 974
- JP-A- 2009 254 348
- RU-C2- 2 281 656
- SOPHIE ST-HILAIRE ET AL: "Fish Offal Recycling by the Black Soldier Fly Produces a Foodstuff High in Omega-3 Fatty Acids", JOURNAL OF THE WORLD AQUACULTURE SOCI, BATON ROUGE, LA. : SOC, US, vol. 38, no. 2, 1 June 2007 (2007-06-01), pages 309-313, XP008151443, ISSN: 0893-8849, DOI: 10.1111/J.1749-7345.2007.00101.X [retrieved on 2007-05-25]

## Description

The invention relates to the field of obtaining nutrients, feed and foodstuffs from insects or worms. In particular, the invention presents a method to convert insects or worms into nutrient streams, encompassing a fat-containing fraction, an aqueous proteinaceous-containing fraction and/or a solid-containing fraction.

In the past decades, there has been a growing interest to use insects and worms as a food source, especially in view of the growth of global population and malnutrition in the developing world. Since insects and worms are rich in proteins and sometimes fats, they represent a relatively high caloric value. Although in some populations it is common to consume insects and worms, e.g. in Africa, Asia, Australia, these are usually eaten as such, be it as a whole or in parts, or used in the preparation of dishes.

However, it is desirable to be able to process insects and worms on an industrial scale to produce nutrients, which subsequently may be used in the preparation of food or feed products.

From several publications, it is known to obtain some particular nutrients from insects, such as proteins or fats.

JP2009254348 A concerns obtaining proteins from bee larvae. Dried larvae are suspended in water, whereto a lypolytic enzyme is added to decompose the lipids. After that, a proteolytic enzyme is added to hydrolyse proteins and the resulting mixture is filtered and the protein is collected. RU 2345139 C2 describes the recovery of chitin from cultivated larvae. WO 2008/091137 concerns an ethanol extract from house fly larvae, which is obtained by drying the larvae, dissolving these in an organic solvent to remove fats and mixing the residue with ethanol to obtain the extract. WO 2011/006276 describes obtaining fatty acids from insect larvae, wherein the fatty acids are extracted using organic solvent.

CN 1 297 691 A discloses a process for producing a protein fraction, a fat fraction and a chitin fraction using housefly maggots (larvae).

It is however not known to fully utilise insects or worms and to convert these into several nutrient streams, such as protein-derived matter, fats and chitin, from which streams the nutrients can optimally and easily be recovered.

An object of the present invention is therefore to provide a method that converts insects or worms into nutrient streams, and preferably into three nutrient streams, being a fat-containing stream, an aqueous stream containing protein-derived matter and yet another stream containing solids such as chitin.

Another object of the invention is to provide a processing method for insects or worms that results in nutrients that are not contaminated with toxic substances and are safe to be used in preparation of various food or feed products and pharmaceuticals.

Yet another object of the invention is to provide a method that is simple, does not require costly equipment or reagents and can easily be scaled up in a large production facility.

Accordingly, the invention provides, in a first aspect, a method to convert insects or worms into nutrient streams, comprising the steps of:
(a) squashing insects or worms thereby obtaining a pulp,
(b) subjecting the pulp to enzymatic hydrolysis obtaining thereby a hydrolysed mixture,
(c) heating the hydrolysed mixture to a temperature of 70-100°C, and
(d) subjecting the mixture to a physical separation step thereby obtaining a fat fraction, an aqueous proteinaceous fraction and a solid-containing fraction.

In another aspect, the present disclosure provides a fat-containing composition, comprising at least 80 wt.% insect or worm fat based on dry weight, of which at least 30 wt.% are saturated fats, the fat comprising at least 7 wt.% lauric acid C12:0, 10-30 wt.% palmitic acid C16:0, and 15-40 wt.% oleic acid C18:1.

In yet another aspect, the invention provides a composition comprising at least 50 wt.% insect or worm protein-derived matter and at most 25 wt.% insect or worm fat based on dry weight, which insect or worm protein-derived matter has a pepsin digestibility of at least 85% as measured by the pepsin-HCl method, elaborated herein-below.

The method according to the invention converts insects or worms into nutrient streams. The term "insects" refers to insects in any development stage, such as adult insects, insect larvae and insect pupae. Preferably, insect larvae or worms are used. A large variety of insects and worms can be used. Preferably, edible insects or edible worms are used. More preferably, the insects are flies, bugs, mosquitos, butterflies, moths, cicadas, termites, bees, ants, wasps, beetles, grasshoppers, or crickets. More preferably, the insects belong to the species: black soldier fly (Hermetia illucens), house fly (Musca domestica), morio worm (Zophobas Morio), mealworm (Tenebrio Molitor) or cricket (Gryllida). In a preferred embodiment, the insects belong to the species black soldier fly. The insects and worms are preferably cultivated, e.g. in an insect farm. The cultivation allows to control and reduces the risks associated with diseases of insects and with the toxicity of insect-derived foodstuffs, e.g. due to the presence insecticides, in contrast to insects harvested in the nature. The conversion of the insects or worms into nutrient streams can suitably be carried out in a reactor vessel.

In step (a) the insects or worms are squashed to obtain a pulp. Preferably, the insects or worms are thereby reduced in size. This results in a homogeneous starting material of viscous consistency. The squashing and reducing in size can conveniently be done in a micro-cutter mill, although other suitable techniques can also be used. During this step, the particle size of the insect or worm remains in the pulp is preferably less than 1 mm (the largest size to be determined using a microscope), more preferably less than 0.5 mm. The particle size can be controlled by selection of a specific knife and plate combination and rotating speed; for example one can use a single or double knife and rotating speed could vary between 1000 and 3000 rpm. A skilled person can find suitable conditions in order to reach a desired particle size. A small particle size is advantageous as it facilitates the enzymatic hydrolysis in the next step.

In step (b) the pulp is subjected to enzymatic hydrolysis. The hydrolysis is preferably carried out at a temperature of 35-65°C. Depending on the enzyme used, it may be necessary to adjust the pH of the pulp. Preferably, the enzymatic hydrolysis is carried out using a protease. The protease can be an acidic protease, a neutral protease or an alkaline protease. If an acidic protease is used, the pH of the pulp may be adjusted to acidic values, preferably to a pH of 3-6. This can be done with any suitable acid and, preferably, citric acid is used. A preferred acidic protease is for example pepsin. If a neutral protease is used, the pH is preferably 6-8. The enzymatic hydrolysis can also be done with an alkaline protease such as papain.

The enzymatic hydrolysis is preferably carried out by continuously stirring in the reactor vessel. It is desired to carry out a complete hydrolysis of the protein present in the insects or worms. Typically, the hydrolysis takes 1-6 hours, preferably 3-5 hours.

At the end of the hydrolysis step, the hydrolysed mixture is heated to a temperature in the range from 70 to 100°C, preferably in the range 80-95°C. This heating step can advantageously be used to stop the enzymatic reaction. The heating also assures that the majority of fats are liquefied in order to prepare the hydrolysed mixture for the following separation step.

In step (d), the hydrolysed mixture is subjected to a physical separation step to obtain nutrient streams. Preferably, the nutrient streams are a fat-containing fraction, an aqueous fraction containing proteinaceous matter and a solid-containing fraction. The physical separation preferably encompasses decanting, centrifuging, or a combination of the two methods.

In a preferred embodiment, first, a fat fraction is separated by decanting, and the remaining mixture is further separated into an aqueous proteinaceous-containing fraction and a solid-containing fraction. However, the fat, proteinaceous and solid-containing fractions can also be obtained in a different order, or simultaneously, e.g. by using a 3-phase decanter. In a preferred embodiment, the physical separation is carried out by using a 3-phase decanter.

The method according to the invention results in a fat fraction, an aqueous proteinaceous fraction and a solid-containing fraction. In this way, the method results in several nutrient streams. Under nutrients streams in the present description streams are understood that contain nutrients, such as fats, proteinaceous material, carbohydrates, minerals and/or chitin. For the purposes of the invention, chitin is also considered a nutrient.

The fat-containing fraction predominantly contains insect or worm fat. Under "predominantly containing", e.g. fat, it is understood that based on the dry weight, the stream contains more fat (on a weight basis) than any other component, or in other words, that fat constitutes the major part of all ingredients based on dry weight. Generally, "predominantly containing" means a content of at least 40 wt.% dry matter, more preferably at least 50 wt.% dry matter. The aqueous proteinaceous fraction predominantly contains protein

The fat-containing fraction obtainable by the method according to the invention, preferably comprises at least 80 wt.%, more preferably 85-95 wt.%, of insect or worm fat based on the dry weight of the fat fraction. The insect or worm fat in the fat fraction comprises at least 30 wt.% and preferably 40-70 wt.% saturated fats, based on the total weight of the fat. The amount of unsaturated fats is 70 wt.% or less, preferably less than 60 wt.% and more preferably 25-55 wt.%, based on the total weight of the fat. The amount of mono unsaturated fatty acids (cis) is preferably from 20 to 45 wt.%, while the amount poly unsaturated fatty acids is preferably from 5 to 20 wt.%. In a preferred embodiment, the insect or worm fat contains at least 7 wt.%, preferably 8-40 wt.%, of lauric acid C12:0. More preferably, the fat contains 20-40 wt.% of lauric acid. The insect or worm fat preferably contains 10-30 wt.% palmitic acid C16:0. Further, the insect or worm fat may further comprise omega-9 fatty acids, preferably in an amount 10-45 wt.%. Under omega-9 fatty acids, the sum of the following acids is understood: oleic acid C18:1, eicosenoic acid C20:1, mead acid C20:3, erucic acid C22:1, nervonic acid C24:1. In particular, the insect or worm fat preferably contains 15-40 wt.% oleic acid C18:1, more preferably, 15-35 wt.%. Omega-6 fatty acids are preferably present in an amount 2-20 wt.%. Under omega-6 fatty acids, the sum of the following acids is understood: linoleic acid C18:2, gamma-linolenic acid C18:3, eicosadienoic acid C20:2, dihomo-gamma-linolenic acid C20:3, arachidonic acid C20:4, docosadioenoic acid C22:2, adrenic acid C22:4, docosapentaenoic acid C22:5, tetracosatetraenoic acid C24:4, tetracosapentaenoic acid C24:5. For example, linoleic acid C18:2 is preferably present in an amount 5-15 wt.%. The amount of trans fatty acids is lower than 0.5 wt.%, preferably lower than 0.2 wt.%. Under trans fatty acids unsaturated fatty acids are meant with at least one carbon-carbon double bond with a trans configuration, e.g. elaidic acid C18:1. The amounts of fatty acids are based on the weight of the insect or worm fat, which is the fat component of the fat-containing fraction. The fatty acid composition is determined by a standard method NEN-EN-ISO 5508+5509, BF3.

Another fraction obtained in the separation step is an aqueous proteinaceous-containing fraction. Proteinaceous is understood to encompass protein, hydrolysed protein, peptides, amino acids and/or other protein-derived compounds obtainable by enzymatic hydrolysis of proteins. The aqueous proteinaceous fraction can further be dried to obtain dried proteinaceous material. This dried material can itself be used as a food or feed ingredient, or it can further be processed, e.g. to isolate amino acids. The aqueous fraction is preferably dried by spray-drying.

The dried proteinaceous material contains at least 50 wt.%, more preferably 55-85 wt.%, insect or worm protein-derived matter and at most 25 wt.%, preferably 3-20 wt.%, insect or worm fat, based on dry weight. The material may further comprise residual moisture, minerals and/or carbohydrates. Under "insect or worm protein" and "insect or worm fat" respectively protein and fat derived from insects or worms are meant. The term "protein-derived matter" is considered synonymous with proteinaceous material. The insect or worm protein-derived matter may thus comprise proteins, hydrolysed proteins, peptides, amino acids. The insect or worm protein-derived matter in the composition above has a pepsin digestibility of at least 85% as determined by a standard "pepsin-HCl" laboratory test such as following the guideline in the Third Commission Directive 72/199/EEC of 27 April 1972. In a preferred embodiment, the proteinaceous material has a protein digestibility of 90-95%. Preferably, the proteinaceous material further contains amino acids, in particular one or more of arginine, lysine, isoleucine, methionine, tryptophan. In a preferred embodiment, the proteinaceous material contains 3-7 wt.% lysine, based on dry weight of the proteinaceous material. In another preferred embodiment, the proteinaceous material further contains minerals such as calcium or phosphorus. Preferably, the calcium content of the proteinaceous material is at least 4,500, more preferably 10,000-30,000 mg/kg, based on dry weight of the proteinaceous material. The phosphorus content of the proteinaceous material is preferably at least 5000 mg/kg, based on dry weight. The calcium and phosphorus content is determined by the OCP-OES method.

The fat fraction of the proteinaceous material preferably contains at least 40 wt.%, more preferably 45-70 wt.% saturated fats and at most 60 wt.%, preferably 25-55 wt.% unsaturated fats based on the weight of the fat fraction. Mono unsaturated fatty acids (cis) are preferably present in an amount 20-45 wt.%, and poly unsaturated fatty acids preferably in an amount 5-20 wt.%. The fat fraction preferably comprises at least 7 wt.%, more preferably 20-45 wt.% of lauric acid C12:0. The fat fraction preferably also comprises 10-30 wt.% palmitic acid C16:0. Further, the fat fraction of the proteinaceous material preferably comprises one or more omega-9 fatty acids in an amount of 10-40 wt.%. In a preferred embodiment, it comprises 15-35 wt.% oleic acid C18:1. The fat fraction also preferably comprises one or more omega-6 fatty acids, in an amount of 5-15 wt.%. The amount of trans fatty acids is preferably lower than 0.5 wt.%. The amounts of fatty acids are based on the weight of the fat fraction of the proteinaceous material. The fat fraction of the proteinaceous material can be isolated for further use.

The remaining solid-containing fraction obtained in the separation step (d), which step encompasses for example decanting or centrifuging, represents a wet pulp, or a suspension. This wet pulp can easily be distinguished from the aqueous proteinaceous fraction. The wet pulp contains solids such as chitin and chitin-derivatives. The wet pulp further preferably comprises proteinaceous matter. This proteinaceous matter has the composition as described herein-above for the aqueous proteinaceous fraction, and has a pepsin digestibility of the protein-derived matter in the range 50-80%, as can be determined by a standard "pepsin-HCl" laboratory test; and particularly by following the guideline in the Third Commission Directive 72/199/EEC of 27 April 1972.

The solid-containing fraction can further be dried to obtain solid material. Preferably, air drying is used. The solid-containing fraction can also be further processed to isolate chitin or proteinaceous material. Chitin is a polysaccharide that can be used in various applications. In food industry, chitin can be used as an additive to thicken and stabilise foods and pharmaceuticals. It can also be used in animal feed as a nutrient source.

The isolated nutrient streams can further be used in the preparation of food or feed, or of food or feed additives, or in pharmaceutical industry. For example, the proteinaceous material and the fat fraction can, respectively, be used in animal feed as a crude protein and a crude fat source. The obtained streams can also be processed further, e.g. to isolate specific ingredients such as hydrolysed protein, amino acids, or specific fatty acids.

The invention is now illustrated in the following, non-limiting example.

### Example

1000 kg fresh larvae of black soldier fly are squashed to obtain insect pulp with an average particle size less than 0.5 mm. The pulp is introduced in a reaction vessel and heated to a temperature of 50°C, whereto 1.1 l of proteolytic enzyme pepsin is added. The pH is lowered to 4 by adding citric acid. The mixture is maintained at 50°C for about 4 hours to allow the hydrolysis reaction. Subsequently, the reaction mixture is heated to 90°C for 1 hour and then brought into a 3-phase decanter. From the decanter three fractions are obtained, being a fat fraction, a "larvae water" and a solid fraction. The "larvae water" contains insect protein derivatives and is spray-dried to obtain insect proteinaceous material.

The composition of the fat fraction (before further separation steps) is given in Table 1. The fatty acids composition of the crude fat is given in Table 2, wherein the percentage is based on the weight of the crude fat. The fatty acids composition was determined by NEN-EN-ISO 5508+5509, BF3 method. The fatty acids are referred to as Cn:m, wherein n is the amount of carbon atoms, and n is the amount of unsaturated carbon-carbon bonds.

**Table 1**

| Component | Content, % |
|---|---|
| Moisture | 7.3 |
| Crude protein (Dumas, N x 6.25) | 0.5 |
| Crude fat (petroleum-ether extraction) | 79.3 |
| Crude fiber (long method) | 4.3 |
| Crude ashes (550°C) | 8.6 |
| Energy value | 2943 kJ/100 g |

**Table 2**

| | |
|---|---|
| Fatty acid | Content (wt.%) |
| C10:0 | 1.3 |
| C12:0 | 38.2 |
| C14:0 | 6.9 |
| C14:1 | 0.3 |
| C16:0 | 16.5 |
| C16:1 | 3.3 |
| C17:0 | 0.1 |
| C18:0 | 2.8 |
| C18:1 | 19.2 |
| C18:1 cis | 0.3 |
| C18:2 | 8.0 |
| C18:3n3 | 1.0 |
| C20:5 | 0.2 |
| trans fatty acids | 0.1 |
| saturated fatty acids | 66.3 |
| mono unsaturated fatty acids | 23.2 |
| poly unsaturated fatty acids | 9.4 |
| unsaturated fatty acids | 32.5 |
| omega-3 fatty acids | 1.3 |
| omega-6 fatty acids | 8.0 |
| omega-9 fatty acids | 19.3 |

The composition of the spray-dried proteinaceous material is given in Table 3. The fat composition of the crude fat fraction of the proteinaceous material is given in Table 4, wherein the percentages refer to percentages by weight based on the total weight of the crude fat fraction. The amino acid composition is given in Table 5, wherein the percentages refer to percentages by weight based on the total weight of the dried proteinaceous material.

**Table 3**

| Component | Content |
|---|---|
| Moisture (dry matter at 103°C), % | 4.4 |
| Crude protein (Dumas, N x 6.25) | 66.2 |
| Crude fat (after pre-extraction and hydrolysis) | 6.4 |
| Crude ashes (550°C) | 7.0 |
| Crude fiber (long method) | 1.5 |
| Energy value, kJ/100 g | 1395 |
| Phosphorus, mg/kg | 5200 |
| Calcium, mg/kg | 4800 |

**Table 4**

| Fatty acid | Content (wt.%) |
|---|---|
| C8:0 | 0.1 |
| C10:0 | 0.9 |
| C12:0 | 32.8 |
| C14:0 | 6.8 |
| C14:1 | 0.2 |
| C15:0 | 0.2 |
| C16:0 | 18.1 |
| C16:1 | 3.4 |
| C17:0 | 0.1 |
| C18:0 | 3.5 |
| C18:1 | 21.5 |
| C18:1 cis | 0.4 |
| C18:2 | 8.9 |
| C18:3n3 | 1.1 |
| C20:1 | 0.1 |
| C20:3n3 | 0.2 |
| C20:5 | 0.2 |
| trans fatty acids | 0.1 |
| saturated fatty acids | 62.8 |
| mono unsaturated fatty acids | 25.7 |
| poly unsaturated fatty acids | 10.4 |
| unsaturated fatty acids | 36.1 |
| omega-3 fatty acids | 1.4 |
| omega-6 fatty acids | 8.9 |
| omega-9 fatty acids | 21.7 |

**Table 5**

| Amino acid | Content (wt.%) |
|---|---|
| Alanine | 5.11 |
| Arginine | 3.29 |
| Asparagic acid | 6.67 |
| Cystine | 0.43 |
| Glutamic acid | 9.08 |
| Glycine | 3.43 |
| Histidine | 2.27 |
| Isoleucine | 3.03 |
| Leucine | 4.60 |
| Lysine | 4.33 |
| Methionine | 1.09 |
| Phenyl-alanine | 2.64 |
| Proline | 4.48 |
| Serine | 2.83 |
| Threonine | 2.99 |
| Tryptophan | 0.87 |
| Tyrosine | 3.87 |
| Valine | 4.38 |

The composition of the air-dried solid fraction is given in Table 6. The fat composition of the crude fat fraction is given in Table 7, wherein the percentages refer to percentages by weight based on the total weight of the crude fat fraction. The amino acid composition is given in Table 8, wherein the percentages refer to percentages by weight based on the total weight of the dried solid fraction. Chitin and chitin-derivatives are comprised in the crude fiber in Table 6.

**Table 6**

| Component | Content |
|---|---|
| Moisture (dry matter at 103°C), % | 10.9 |
| Crude protein (Dumas, N x 6.25) | 53.2 |
| Crude fat (after pre-extraction and hydrolysis) | 11.4 |
| Crude ashes (550°C) | 5.8 |
| Crude fiber (long method) | 20.5 |
| Energy value, kJ/100 g | 1331 |
| Phosphorus, mg/kg | 5100 |
| Calcium, mg/kg | 11000 |

**Table 7**

| Fatty acid | Content (wt.%) |
|---|---|
| C8:0 | 0.1 |
| C10:0 | 1.3 |
| C12:0 | 33.3 |
| C14:0 | 6.0 |
| C14:1 | 0.3 |
| C15:0 | 0.2 |
| C16:0 | 16.4 |
| C16:1 | 3.5 |
| C17:0 | 0.1 |
| C18:0 | 3.2 |
| C18:1 | 21.4 |
| C18:1 cis | 0.4 |
| C18:2 | 9.1 |
| C18:3n3 | 1.1 |
| C20:1 | 0.1 |
| C20:3n3 | 0.2 |
| C20:5 | 0.2 |
| trans fatty acids | 0.2 |
| saturated fatty acids | 61.2 |
| mono unsaturated fatty acids | 25.7 |
| poly unsaturated fatty acids | 10.6 |
| unsaturated fatty acids | 36.3 |
| omega-3 fatty acids | 1.5 |
| omega-6 fatty acids | 9.1 |
| omega-9 fatty acids | 21.5 |

**Table 8**

| Amino acid | Content (wt.%) |
|---|---|
| Alanine | 3.71 |
| Arginine | 2.33 |
| Asparagic acid | 4.38 |
| Cystine | 0.34 |
| Glutamic acid | 5.01 |
| Glycine | 3.01 |
| Histidine | 1.53 |
| Isoleucine | 2.49 |
| Leucine | 4.02 |
| Lysine | 3.01 |
| Methionine | 0.91 |
| Phenyl-alanine | 2.08 |
| Proline | 3.15 |
| Serine | 2.24 |
| Threonine | 2.09 |
| Tryptophan | 0.78 |
| Tyrosine | 3.12 |
| Valine | 3.29 |

## Claims

1. Method to convert insects or worms into nutrient streams, comprising the steps of:
(a) squashing insects or worms thereby obtaining a pulp,
(b) subjecting the pulp to enzymatic hydrolysis obtaining thereby a hydrolysed mixture,
(c) heating the hydrolysed mixture to a temperature of 70-100°C, and
(d) subjecting the mixture to a physical separation step thereby obtaining a fat fraction, an aqueous proteinaceous fraction and a solid-containing fraction.

2. The method according to claim 1, wherein the insects are adult insects, insect larvae or insect pupae.

3. The method according to claim 1 or 2, wherein the insects belong to the species house fly, black soldier fly, morio worm, mealworm or cricket.

4. The method according to any one of the preceding claims, wherein the pulp is hydrolysed using a protease at a temperature of 35-65°C.

5. The method according to claim 4, wherein the protease is an acidic protease and the pulp is acidified to a pH of 3-6.

6. The method according to claim 4, wherein the protease is used at a pH 6-8.

7. The method according any one of the preceding claims, wherein the physical separation encompasses decanting and/or centrifuging.

8. The method according to any one of the preceding claims, wherein the aqueous proteinaceous fraction and/or the solid-containing fraction is further dried.

9. The method according to claim 8, wherein the aqueous proteinaceous fraction is spray-dried.

10. The method according to any one of the preceding claims, wherein the particle size of insect or worm remains in the pulp is less than 1 mm.

11. A composition comprising at least 50 wt.% insect or worm protein-derived matter and at most 25 wt.% insect or worm fat based on dry weight, which insect or worm protein-derived matter has a pepsin digestibility of at least 85%, preferably 90-95%, as measured by the pepsin-HCl method.

12. The composition according to claim 11, wherein the insect or worm fat comprises at least 40 wt.%, preferably 45-70 wt.% saturated fats based on the weight of the fat fraction.

13. The composition according to claim 11 or 12, further comprising at least 4,500 mg/kg Ca based on dry weight.

14. The composition according to any one of claims 11-13, comprising 55-85 wt.% insect or worm protein-derived matter.

15. The composition according to any one of claims 11-14, further comprising one or more of amino acids selected from the group consisting of arginine, lysine, isoleucine, methionine, and tryptophan.

16. The composition according to claim 15, comprising 3-7 wt.% lysine based on the dry weight of the composition.

## Patentansprüche

1. Verfahren zum Umwandeln von Insekten oder Würmern in Nährstoffströme, das die Schritte aufweist:
(a) Zerquetschen von Insekten oder Würmern, wodurch ein Brei erhalten wird,
(b) Unterwerfen des Breis einer enzymatischen Hydrolyse, wodurch eine hydrolysierte Mischung erhalten wird,
(c) Erwärmen der hydrolysierten Mischung auf eine Temperatur von 70-100°C, und
(d) Unterwerfen der Mischung einem physikalischen Trennschritt, wodurch eine Fettfraktion, eine wässrige proteinhaltige Fraktion und eine feststoffhaltige Fraktion erhalten werden.

2. Verfahren nach Anspruch 1, wobei die Insekten erwachsene Insekten, Insektenlarven oder Insektenpuppen sind.

3. Verfahren nach Anspruch 1 oder 2, wobei die Insekten zur Gattung Stubenfliege, Soldatenfliege, Larve des Großen Schwarzkäfers, Mehlwurm oder Grille gehören

4. Verfahren nach einem der vorhergehenden Ansprüche, wobei der Brei unter Verwendung einer Protease bei einer Temperatur von 35-65°C hydrolysiert wird.

5. Verfahren nach Anspruch 4, wobei die Protease eine saure Protease ist und der Brei auf einen pH-Wert von 3-6 angesäuert wird.

6. Verfahren nach Anspruch 4, wobei die Protease bei einem pH-Wert von 6-8 verwendet wird.

7. Verfahren nach einem der vorhergehenden Ansprüche, wobei die physikalische Trennung Dekantieren und/oder Zentrifugieren umfasst.

8. Verfahren nach einem der vorhergehenden Ansprüche, wobei die wässrige proteinhaltige Fraktion und/oder die feststoffhaltige Fraktion ferner getrocknet werden.

9. Verfahren nach Anspruch 8, wobei die wässrige proteinhaltige Fraktion sprühgetrocknet wird.

10. Verfahren nach einem der vorhergehenden Ansprüche, wobei die Teilchengröße der Insekten- oder Wurmüberreste im Brei kleiner als 1 mm ist.

11. Zusammensetzung, die mindestens 50 Gew.-% von Insekten- oder Wurmprotein stammendes Material und höchstens 25 Gew.-% Insekten- oder Wurmfett bezogen auf das Trockengewicht aufweist, wobei das von Insekten- oder Wurmprotein stammende Material eine durch das Pepsin-HCl-Verfahren gemessene Pepsinverdaubarkeit von mindestens 85%, vorzugsweise 90-95% aufweist.

12. Zusammensetzung nach Anspruch 11, wobei das Insekten- oder Wurmfett mindestens 40 Gew.-%, vorzugsweise 45-70 Gew.-% gesättigte Fette bezogen auf das Gewicht der Fettfraktion aufweist.

13. Zusammensetzung nach Anspruch 11 oder 12, die ferner mindestens 4500 mg/kg Ca bezogen auf das Trockengewicht aufweist.

14. Zusammensetzung nach einem der Ansprüche 11 bis 13, die 55-85 Gew.-% von Insekten- oder Wurmprotein stammendes Material aufweist.

15. Zusammensetzung nach einem der Ansprüche 11 bis 14, die ferner eine oder mehrere Aminosäuren aufweist, die aus der Gruppe ausgewählt sind, die aus Arginin, Lysin, Isoleucin, Methionin und Tryptophan besteht.

16. Zusammensetzung nach Anspruch 15, die 3-7 Gew.-% Lysin bezogen auf das Trockengewicht der Zusammensetzung aufweist.

## Revendications

1. Procédé de conversion d'insectes ou de vers en flux nutritifs, comprenant les étapes suivantes :
(a) écrasement d'insectes ou de vers afin d'obtenir une pulpe,
(b) soumission de la pulpe à une hydrolyse enzymatique pour obtenir un mélange hydrolysé,
(c) chauffage du mélange à une température comprise entre 70 et 100 °C, et
(d) soumission du mélange à une étape de séparation physique permettant d'obtenir une fraction lipidique, une fraction protéinique aqueuse et une fraction contenant des solides.

2. Procédé selon la revendication 1, où les insectes sont des insectes adultes, des larves d'insectes ou des nymphes d'insectes.

3. Procédé selon la revendication 1 ou la revendication 2, où les insectes appartiennent aux espèces : mouche, mouche soldat noire, vers de farine géants, vers de farine ou criquet.

4. Procédé selon l'une des revendications précédentes, où la pulpe est hydrolysée au moyen d'une protéase à une température comprise entre 35 et 65 °C.

5. Procédé selon la revendication 4, où la protéase est une protéase acide et la pulpe est acidifiée à un pH comprise entre 3 et 6.

6. Procédé selon la revendication 4, où la protéase est utilisée à un pH compris entre 6 et 8.

7. Procédé selon l'une des revendications précédentes, où la séparation physique englobe la décantation et/ou la centrifugation.

8. Procédé selon l'une des revendications précédentes, où la fraction protéinique aqueuse et/ou la fraction contenant des solides sont séchées ensuite.

9. Procédé selon la revendication 8, où la fraction protéinique aqueuse est séchée par pulvérisation.

10. Procédé selon l'une des revendications précédentes, où la taille des particules des restes d'insectes ou de vers dans la pulpe est inférieure à 1 mm.

11. Composition, comprenant au moins 50 % en poids de matière dérivée de protéines d'insectes ou de vers et au plus 25 % en poids de graisses d'insectes ou de vers, par rapport au poids à sec, ladite matière dérivée de protéines d'insectes ou de vers présentant une digestibilité de pepsine d'au moins 85 %, préférentiellement de 90 à 95%, telle que mesurée au moyen de la méthode pepsine-HCl.

12. Composition selon la revendication 11, où les graisses d'insectes ou de vers comprennent au moins 40 % en poids, préférentiellement de 45 à 70% en poids de graisses saturées, sur la base du poids de la fraction lipidique.

13. Composition selon la revendication 11 ou la revendication 12, comprenant en outre au moins 4500 mg/kg de Ca par rapport au poids à sec.

14. Composition selon l'une des revendications 11 à 13, comprenant de 55 à 85 % en poids de matière dérivée de protéines d'insectes ou de vers.

15. Composition selon l'une des revendications 11 à 14, comprenant en outre un ou plusieurs acides aminés choisis dans le groupe constitué par l'arginine, la lysine, l'isoleucine, la méthionine et le tryptophane.

16. Composition selon la revendication 15, comprenant de 3 à 7 % en poids de lysine par rapport au poids à sec de la composition.
